# EUROPEAN PATENT APPLICATION

(11) **EP 3 257 483 A1**
(43) Date of publication of application: **20.12.2017**
(21) Application number: 15881846.8
(22) Date of filing: 10.12.2015
(51) Int. Cl.: A61F 5/56

(54) **IMPLANTABLE LINGUAL SEPTUM FASCIA TRACTION DEVICE AND IMPLANTATION METHOD**

(30) Priority: 09.02.2015 CN 201510083183
(71) Applicant: Zhou, Xing, Guangdong 510663 (CN); Zhang, Xiangmin, Guangzhou, Guangdong 510663 (CN)
(72) Inventor: Zhou, Xing, Guangdong 510663 (CN); Zhang, Xiangmin, Guangzhou, Guangdong 510663 (CN)
(74) Representative: Boult Wade Tennant
(86) International application number: PCT/CN2015/096891
(87) International publication number: WO 2016/127697

(57) **Abstract**

An implanted tongue septum fascia retraction device of the present application includes a fascia fixer, a pull line and a mandible fixer. The fascia fixer is fixed to a tongue septum fascia by means of a fixing clamp, a fixing hook or a fixing ring, and the mandible fixer is fixed to a mandible. One end of the pull line is connected to the fascia fixer, and the other end of the pull line is fixed to the mandible fixer. By adjusting the tightness of a pull line, a tongue septum fascia may be retracted forwards, thus pulling a falling tongue base forwards, opening an obstructed upper airway, and fulfilling the aim of treating snoring and obstructive sleep apnea/hypopnea syndrome (OSA) caused by glossoptosis.

## Description

### TECHNICAL FIELD

The present application relates to a retraction device that is implanted into the tongue body of a human body to retract the tongue base and/or the tongue dorsum forwards and an implantation method, and more particularly to an implanted tongue septum fascia retraction device for treating snoring or adult obstructive sleep apnea/hypopnea syndrome (OSA) caused by glossoptosis and an implantation method.

### BACKGROUND

Adult OSA is a sleep breathing disorder with clinical features of snoring and apnea caused by upper airway collapse and obstruction during sleep. The major hazard of OSA is that frequent sleep apnea and hypopnea result in lowered oxygen saturation levels during sleep for a long time, causing a series of pathologic changes in the human body, thus becoming the cause of various systemic diseases (such as diabetes, hypertension, coronary heart disease, and cerebrovascular accident). According to statistics, currently the morbidity of OSA is up to 2%-4% among people, and is extremely high among the middle-aged, which seriously affects the health and quality of life. Therefore, it has been listed by the World Health Organization as one of the major diseases that affect the health and quality of life of people.

As for the pathogenesis of OSA, it is generally considered that the main cause is that, pharyngeal muscles for maintaining the upper airway open relax during sleep, resulting in soft tissue collapse and obstruction, and the glossopharyngeal portion is an important plane where an OSA patient suffers from soft tissue collapse and obstruction.

As for the obstruction of the glossopharyngeal portion caused by glossoptosis, according to current tongue retraction methods, tongue muscles are retracted using a device, and since the own tension bearing capacities of the tongue muscles are limited, the muscles are easily cut during long-term retraction. Therefore, although these methods have a certain OSA treatment effect, long-term treatment effects are not ideal, so the methods need to be improved.

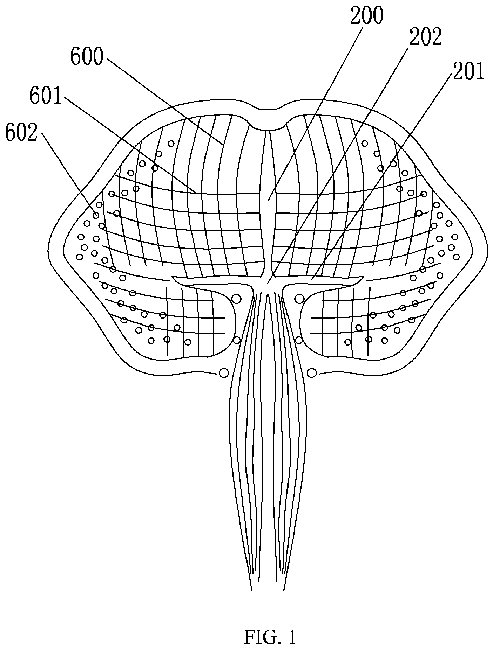

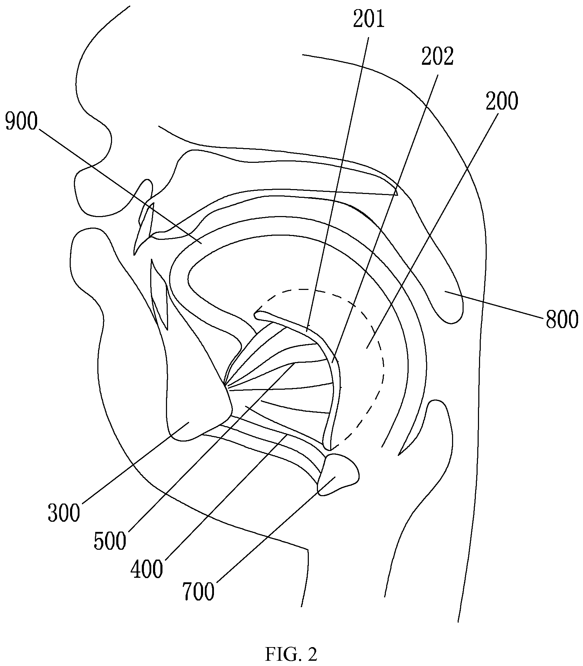

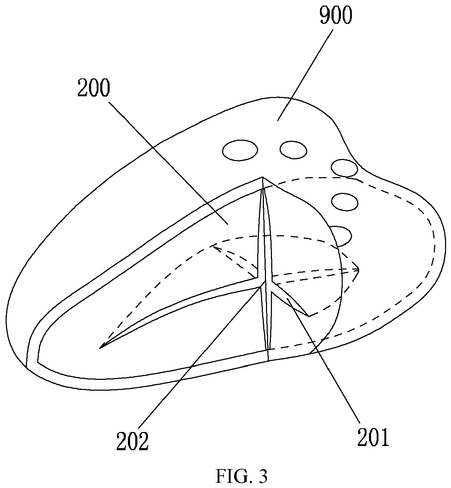

It is discovered by anatomical studies that a transversely extending fascia, hereinafter referred to as a tongue transverse fascia, exists at a connected and attached part of an intrinsic tongue muscle and an extrinsic tongue muscle; a longitudinally extending tongue septum fascia, hereinafter referred to as a tongue septum fascia, exists at a connected and attached part of intrinsic tongue muscles on the left and right sides of a tongue middle line; the tongue transverse fascia and the tongue septum fascia are distributed crisscross, and an intersection of the two fascias presents an obvious white tough tissues anatomically, hereinafter referred to as a tongue septum white line; and the intersection of the two fascias, namely the tongue septum white line, may bear a larger retraction force, and with reference to FIG. 1 to FIG. 3, experiments show that the tongue septum white line may bear a tension of 2Kg without injuries. For example, the tongue septum fascia or the tongue septum white line is retracted to the front lower side of the tongue by means of a special tool, the effect of retraction in the same direction of musculus genioglossus may be achieved, airways of the posterior part of the tongue and the posterior part of the palate can be effectively opened, and the aim of treating snoring and OSA is fulfilled.

The present application is exactly an implanted medical device designed according to anatomical features of the tongue of the human body and dedicated to retraction of the tongue septum fascia. By retracting the tongue septum fascia or the tongue septum white line to the front lower side of the tongue and opening the airways of the posterior part of the tongue and the posterior part of the palate, the aim of treating snoring and OSA is fulfilled.

### SUMMARY

The core of the present application lies in that: an implanted medical instrument, namely an implanted tongue septum fascia retraction device 100, is designed. By means of the feature that a tongue septum fascia 200 is capable of bearing a large retraction force, a fascia fixer 1 is fixed to the tongue septum fascia 200, a mandible fixer 3 is fixed to a mandible 300, the fascia fixer 1 is connected to the mandible fixer 3 by means of a pull line 2, one end of the pull line 2 is connected to the fascia fixer 1, and the other end of the pull line 2 is connected to the mandible fixer 3. By adjusting the length of the pull line 2, the tongue septum fascia 200 is retracted forwards, a falling tongue base is pulled forwards, an obstructed upper airway is opened, and therefore the aim of treating snoring and OSA caused by glossoptosis is fulfilled.

An implanted tongue septum fascia retraction device is characterized in that:
A, the implanted tongue septum fascia retraction device 100 includes: a fascia fixer 1, a pull line 2 and a mandible fixer 3, the fascia fixer 1 being a fixing clamp 11 capable of clamping a tongue septum fascia 200 or a fixing hook 12 capable of passing through the tongue septum fascia 200 or a fixing ring 13 capable of passing through the tongue septum fascia 200, the pull line 2 being a linear object capable of being implanted into a human body for a long time, the mandible fixer 3 being a fixing mechanism capable of being fixed to a mandible 300; and
B, one end of the pull line 2 is connected to the fascia fixer 1, and the other end of the pull line 2 is connected to the mandible fixer 3.

The fascia fixer 1 is fixed to the tongue septum fascia 200 by means of the fixing clamp 11, the fixing hook 12 or the fixing ring 13, one end of the pull line 2 is connected to the fascia fixer 1, and the other end of the pull line 2 is fixed to the mandible 300 by means of the mandible fixer 3. By adjusting the tightness of the pull line 2, the tongue septum fascia 200 may be retracted forwards, thus pulling the falling tongue base forwards.

The implanted tongue septum fascia retraction device 100 further includes an excess force protection mechanism 4. The excess force protection mechanism 4 is disposed at the end of the mandible fixer 3, one end of the excess force protection mechanism 4 is connected to the mandible fixer 3, and the other end of the excess force protection mechanism 4 is connected to the pull line 2. Or, the excess force protection mechanism 4 is disposed at the end of the fascia fixer 1, one end of the excess force protection mechanism 4 is connected to the fascia fixer 1, and the other end of the excess force protection mechanism 4 is connected to the pull line 2. Or, the excess force protection mechanism 4 is disposed in the middle of the pull line 2, and two ends of the excess force protection mechanism 4 are connected to the pull line 2 separately.

The falling tongue body of a patient suffering from snoring and OSAS caused by glossoptosis is usually retracted by virtue of a retraction force of less than 200 g, an airway of a glossopharyngeal portion may be opened to 10-15 mm, and a good treatment effect is achieved accordingly. During saliva swallowing or other swallowing activities, the force of backward movement of the tongue body is usually larger than 500 g. To prevent discomfort caused by excessive influences and limits on the movement of the tongue body in a swallowing process, the excess force protection mechanism 4 needs to be designed, and the excess force protection mechanism 4 is capable of achieving a buffer function, enables the movement of the tongue body to be buffered, improves the comfort, and protects tongue tissues from being injured accidentally.

The excess force protection mechanism 4 includes an elastic mechanism 41.

The elastic mechanism 41 adopts a spring structure.

Further, the spring structure is a spiral spring structure. During saliva swallowing or other swallowing activities, the force of backward movement of the tongue body is usually large. When the force generated by the swallowing activities is larger than the deformation force of the elastic mechanism 41, the elastic mechanism 41 deforms, a buffer function is achieved, the comfort may be improved, and the tongue tissues are protected from being injured accidentally.

The excess force protection mechanism 4 further includes a housing 42.

The elastic mechanism 41 of the excess force protection mechanism 4 is disposed in the housing 42 of the excess force protection mechanism 4. By disposing the elastic mechanism 41 in the housing 42, the influence on peripheral biological tissues in a spring deformation process may be reduced.

A concave-convex engagement structure is formed between a left arm 11-1 and a right arm 11-2 of the fixing clamp 11 of the fascia fixer 1.

The fascia fixer 1 may be connected to the fascia 200 by means of the dentate concave-convex engagement structure formed between the left arm 11-1 and the right arm 11-2 of the fixing clamp 11. During use, fixed connection may be completed by directly clamping the fascia 200 by the fixing clamp 11, thus achieving a convenient use effect.

The fixing hook 12 of the fascia fixer 1 is a C-shaped hook or a U-shaped hook. The fixing hook 12 is made from a medical titanium metal wire, the diameter of the fixing hook 12 is larger than 1 mm, and the fixing hook 12 is usually made from a titanium metal wire having a diameter of 2.5 mm. The fixing hook 12 may conveniently hook the tongue septum fascia 200, thus avoiding pressure on the tongue septum fascia 200. Moreover, the fixing hook 12, hooking the tongue septum fascia 200, may flexibly move so as to be suitable for irregular operation of the tongue body. To prevent the fixing hook 12 from slipping out of the tongue septum fascia 200, the shape of the fixing hook 12 may also be designed as a U shape or other more complicated shapes.

The fixing ring 13 of the fascia fixer 1 includes a left arm 13-1 and a right arm 13-2, a positioning hole 13-2-1 is provided on the right arm 13-2, a puncture rod 13-1-1 is disposed on the left arm 13-1, and the puncture rod 13-1-1 passes through the fascia 200 and then matches the positioning hole 13-2-1, so as to form a closed ring structure.

A groove hole 13-1-1-1 may be provided on the puncture rod 13-1-1, thus making it convenient to insert the puncture rod 13-1-1 into the positioning hole 13-2-1 to be inlaid and fixed. The puncture rod 13-1-1 may be further provided with a positioning convex step 13-1-1-2, the height (usually around 1 mm) of the positioning convex step 13-1-1-2 being slightly larger than the thickness of the tongue septum fascia 200, thus preventing the tongue septum fascia 200 from being over-pressed after the left arm 13-1 and the right arm 13-2 of the fixing ring 13 are closed, and maintaining the biological activity of the tongue septum fascia 200.

The fixing ring 13 may be of a circular ring structure. an elliptical ring structure or a polygonal ring structure.

The pull line 2 is an elastic line or a non-elastic line that is implanted into a human body for a long time.

The mandible fixer 3 is a bone nail 31. The bone nail 31 may be of a threaded structure, thus achieving the effects of convenience in fixing, safety and reliability.

The mandible fixer 3 is a fixing mechanism 32 capable of being fixed to the mandible 300, the fixing mechanism 32 including a housing 32-1 and a line fixing mechanism 32-2 capable of fixing the pull line.

The fixing mechanism 32 includes an adjusting device 32-3 capable of adjusting the tightness of the pull line 2, the adjusting device 32-3 being a threaded adjusting mechanism, a rotary shaft type adjusting mechanism or a slide block type adjusting mechanism. By adjusting the adjusting device 32-3, the tightness of the pull line 2 may be adjusted, thereby adjusting a retraction action exerted on the tongue septum fascia 200 by the implanted tongue septum fascia retraction device of the present application.

The implanted tongue septum fascia retraction device 100 includes the fascia fixer 1, the pull line 2 and the mandible fixer 3. The fascia fixer 1 is fixed to the tongue septum fascia 200 by means of the fixing clamp 11, the fixing hook 12 or the fixing ring 13, and the mandible fixer 3 is fixed to the mandible 300 by means of the bone nail 31 or the fixing mechanism 32. One end of the pull line 2 is connected to the fascia fixer 1, and the other end of the pull line 2 is fixed to the mandible fixer 3. By adjusting the tightness of the pull line 2, the tongue septum fascia 200 may be retracted forwards, thus pulling the falling tongue base forwards, opening the obstructed upper airway, and fulfilling the aim of treating snoring and OSA caused by glossoptosis.

An implantation method of the present application:

When a lower edge of a mandible 300 is selected as an entry point for implanting the implanted tongue septum fascia retraction device of the present application, the implantation method includes:
first step: transversely incising the skin and subcutaneous tissues in the center of a front lower edge of the mandible along dermatoglyph under local anesthesia or general anesthesia;
second step: separating, after hemostasis, the subcutaneous tissues, and separating left and right musculus geniohyoideus 400 and left and right musculus genioglossus 500 along a center line using a pair of hemostatic forceps or other separators, a longitudinally extending white tongue septum fascia (200) being seen at a junction of the left and right musculus genioglossus 500, wherein in order to facilitate mounting of a fascia fixer 1, it is suggested that the white tongue septum fascia 200 having a length of about 4 cm is separated;
third step: putting the fascia fixer 1 connected with a pull line 2 to the tongue septum fascia 200 through an incision at the lower edge of the mandible 300 using a special tool;
fourth step: fixing the implanted tongue septum fascia retraction device 100 to the tongue septum fascia 200 by means of a fixing clamp 11, a fixing hook 12 or a fixing ring 13 on the fascia fixer 1;
fifth step: fixing a mandible fixer 3 to the mandible 300 by means of a bone nail 31 or a fixing mechanism 32 capable of being fixed to the mandible 300; and
sixth step: connecting the pull line 2 to the mandible fixer 3, adjusting the tightness of the pull line, and suturing the incision, so as to complete implantation of the implanted tongue septum fascia retraction device 100.

When the implanted tongue septum fascia retraction device of the present application is selected to be implanted from an oral cavity, the implantation method includes:
first step: transversely incising the mucosa at lower gingival sulcus of the lower lip under local anesthesia or general anesthesia;
second step: separating and exposing front bones of the mandible, and drilling a through hole from a lower edge of the mandible 300 to a center point of the lower tooth socket using a tool;
third step: putting, after hemostasis, a fascia fixer 1 connected with a pull line 2 to the tongue septum fascia 200 through the through hole using a special tool;
fourth step: fixing the implanted tongue septum fascia retraction device 100 to the tongue septum fascia 200 by means of a fixing clamp 11, a fixing hook 12 or a fixing ring 13 on the fascia fixer 1;
fifth step: fixing a mandible fixer 3 to the mandible 300 by means of a bone nail 31 or a fixing mechanism 32 capable of being fixed to the mandible 300; and
sixth step: connecting the pull line 2 to the mandible fixer 3, adjusting the tightness of the pull line, and suturing the incision, so as to complete implantation of the implanted tongue septum fascia retraction device 100.

### BRIEF DESCRIPTION OF THE DRAWINGS

FIG. 1 is a schematic distribution view of a tongue septum fascia and a tongue transverse fascia of a coronal position of the tongue;
FIG. 2 is a schematic distribution view of a tongue septum fascia and a tongue transverse fascia of a sagittal position of the tongue;
FIG. 3 is a schematic distribution view of a tongue septum fascia and a tongue transverse fascia in a three-dimensional cross-sectional view of the tongue;
FIG. 4 is a schematic structural view of a fixing clamp type implanted tongue septum fascia retraction device according to the present application;
FIG. 4-1 is a schematic structural view of the closed implanted tongue septum fascia retraction device of FIG. 4;
FIG. 4-2 is a cross-sectional view of FIG. 4-1;
FIG. 4-3 is a view depicting the working principle of FIG. 4;
FIG. 5 is a schematic structural view of a C-shaped fixing hook type implanted tongue septum fascia retraction device according to the present application;
FIG. 5-1 is a cross-sectional view of FIG. 5;
FIG. 5-2 is a view depicting the working principle of FIG. 5;
FIG. 6 is a schematic structural view of a U-shaped fixing hook type implanted tongue septum fascia retraction device according to the present application;
FIG. 6-1 is a cross-sectional view of FIG. 6;
FIG. 7 is a schematic structural view of a fixing ring type implanted tongue septum fascia retraction device according to the present application;
FIG. 7-1 is a schematic structural view of the closed implanted tongue septum fascia retraction device of FIG. 7;
FIG. 7-2 is a cross-sectional view of FIG. 7-1;
FIG. 7-3 is a view depicting the working principle of FIG. 7;
FIG. 8 is a schematic structural view of an implanted tongue septum fascia retraction device having an excess force protection mechanism disposed at the end of a fascia fixer according to the present application;
FIG. 8-1 is a schematic structural view of the closed implanted tongue septum fascia retraction device of FIG. 7;
FIG. 8-2 is a cross-sectional view of FIG. 7-1;
FIG. 9 is a schematic structural view of an implanted tongue septum fascia retraction device having an excess force protection mechanism disposed in the middle of a pull line according to the present application;
FIG. 9-1 is a schematic structural view of the closed implanted tongue septum fascia retraction device of FIG. 9;
FIG. 9-2 is a cross-sectional view of FIG. 9-1;
FIG. 10 is a schematic structural view of an implanted tongue septum fascia retraction device having an adjustable mandible fixer according to the present application;
FIG. 10-1 is a schematic structural view of the closed implanted tongue septum fascia retraction device of FIG. 10;
FIG. 10-2 is a view depicting the working principle of the implanted tongue septum fascia retraction device of FIG. 10;
FIG. 11 is a schematic structural view and a view depicting the working principle of an implanted tongue septum fascia retraction device having a suspended mandible fixer according to the present application;
FIG. 12 is a schematic structural view of an implanted tongue septum fascia retraction device having an adjustable mandible fixer including an excess force protection mechanism according to the present application;
FIG. 12-1 is a schematic structural view of the closed implanted tongue septum fascia retraction device of FIG. 12.

In the above drawings:
100. implanted tongue septum fascia retraction device; 200. tongue septum fascia, 201. tongue transverse fascia, 202. tongue septum white line; 300. mandible, 400. musculus geniohyoideus, 500. musculus genioglossus, 600. vertical muscle, 601. tongue transverse muscle, 602. tongue longitudinal muscle; 700. tongue bone, 800. soft palate, 900. tongue
1. fascia fixer, 2. pull line, 3. mandible fixer
11. fixing clamping type fascia fixer; 11-0. through hole, 11-1. left arm of fixing clamp, 11-1-1. positioning convex step/hole on left arm of fixing clamp; 11-2. right arm of fixing clamp, 11-2-1. positioning convex step/hole on right arm of fixing clamp; 11-3. line fixing mechanism, 11-3-1. line fixing head, 11-3-2. housing, 11-3-3. connecting bolt
12. fixing hook type fascia fixer; 12-1. line fixing mechanism of fixing hook, 12-1-1. fixing head, 12-1-2. housing
13. fixing ring type fascia fixer; 13-0. through hole; 13-1. left arm of fixing ring, 13-1-1. puncture rod on left arm of fixing ring, 13-1-2. positioning convex step/hole on left arm of fixing ring, 13-1-1-1. groove hole on puncture rod, 13-1-1-2. positioning convex step on puncture rod; 13-2. right arm of fixing ring, 13-2-1. positioning hole on right arm of fixing ring, 13-2-2. positioning convex step/hole on right arm of fixing ring; 13-3. line connecting mechanism, 13-3-1. line fixing head, 13-3-2. housing, 13-3-3. connecting bolt
31. bone nail type mandible fixer, 32. fixing mechanism type mandible fixer, 33. line fixing mechanism
31-1. mounting groove, 31-2. positioning sheet, 31-3. line fixing head
32-1. housing of fixing mechanism type mandible fixer, 32-1-1. end cover of housing, 32-1-2. main body of housing; 32-2. pull line fixing mechanism, 32-2-1. line fixing head; 32-3. pull line tension adjusting mechanism, 32-3-1. rotating screw, 32-3-2. screw slide block
33-1. line fixing head
4. excess force protection mechanism; 41. elastic mechanism of excess force protection mechanism; 42. housing of excess force protection mechanism, 42-1. through hole, 42-2. positioning concave groove; 43. slide block; 44. line fixing head.

### DESCRIPTION OF EMBODIMENTS

### Embodiment 1: a fixing clamp type implanted tongue septum fascia retraction device of the present application

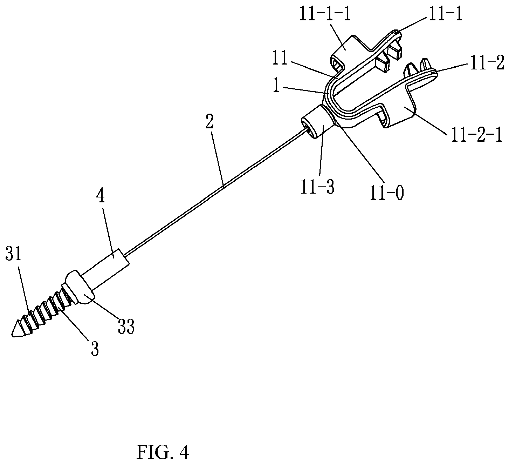

With reference to FIG. 4 to FIG. 4-3, the implanted tongue septum fascia retraction device 100 in this embodiment includes a fascia fixer 1, a pull line 2 and a mandible fixer 3. In this embodiment, the implanted tongue septum fascia retraction device 100 further includes an excess force protection mechanism 4. The excess force protection mechanism 4 is disposed at the end of the mandible fixer 3, one end of the excess force protection mechanism 4 is connected to the mandible fixer 3, and the other end of the excess force protection mechanism 4 is connected to the pull line 2.

The falling tongue body of a patient suffering from snoring and OSAS caused by glossoptosis is usually retracted by virtue of a retraction force of less than 200 g, an airway of a glossopharyngeal portion may be opened to 10-15 mm, and a good treatment effect is achieved accordingly. During saliva swallowing or other swallowing activities, the force of backward movement of the tongue body is usually larger than 500 g. To prevent discomfort caused by excessive influences and limits on the movement of the tongue body in a swallowing process, the excess force protection mechanism 4 needs to be designed, and the excess force protection mechanism 4 is capable of achieving a buffer function, enables the movement of the tongue body to be buffered, improves the comfort, and protects tongue tissues from being injured accidentally.

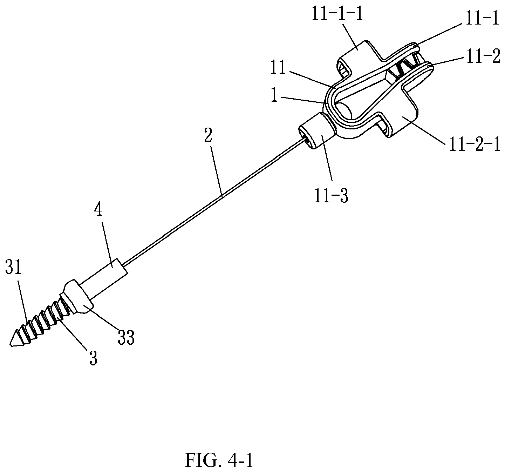

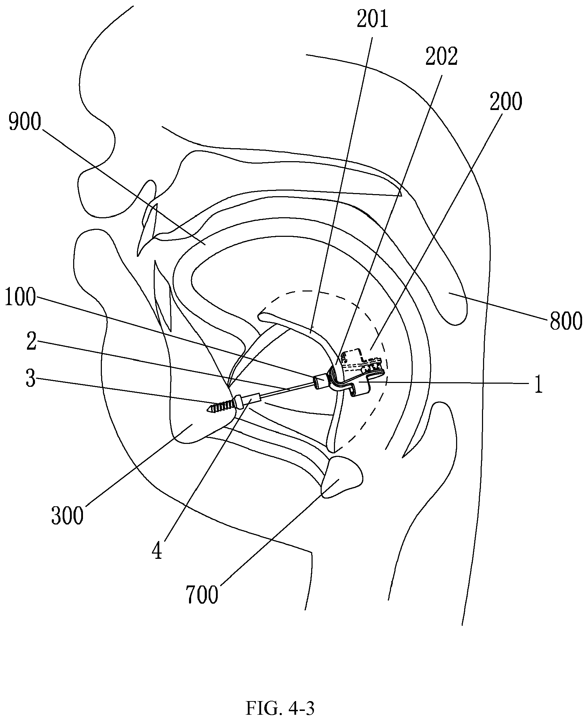

In this embodiment, the fascia fixer 1 is a fixing clamp 11. The fixing clamp 11 includes a left arm 11-1 and a right arm 11-2. A dentate concave-convex engagement structure is formed between the left arm 11-1 and the right arm 11-2 and configured to clamp a tongue septum fascia 200, with reference to FIG. 4-1 to FIG. 4-3.

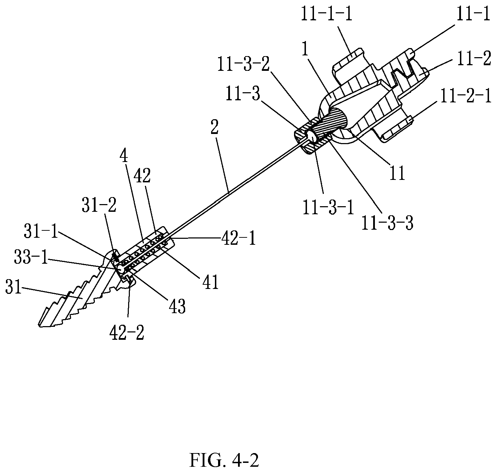

In order to facilitate connection between the pull line 2 and the fascia fixer 1, in this embodiment, a through hole 11-0 and a pull line fixing mechanism 11-3 for fixing the pull line 2 are further provided at a junction of the left arm 11-1 and the right arm 11-2, the pull line fixing mechanism 11-3 including a line fixing head 11-3-1, a housing 11-3-2 and a connecting bolt 11-3-3. After passing through the through hole of the housing 11-3-2, the end part of the pull line 2 is riveted and fixed to the metal line fixing head 11-3-1. After passing through the through hole 11-0, the connecting bolt 11-3-3 is connected to the housing 11-3-2 in a threaded connection manner. The connection has the advantages that the metal spherical line fixing head 11-3-1 can more flexibly adjust a retraction direction so as to be suitable for irregular movement of the tongue body, with reference to FIG. 4-2 and FIG. 4-3.

Besides, in order to conveniently deliver the fixing clamp 11 of the fascia fixer 1 to the tongue septum fascia 200, a positioning convex step/hole 11-1-1 and a positioning convex step/hole 11-2-1 are provided on the left arm 11-1 and the right arm 11-2 respectively and configured to match a special surgical delivery instrument and perform positioning. The fixing clamp 11 is kept in an open state, after passing through a tongue transverse fascia 201, the left arm 11-1 and the right arm 11-2 are placed on two sides of the tongue septum fascia 200, and the tongue septum fascia 200 is kept between the left arm 11-1 and the right arm 11-2, and then matches the special surgical delivery instrument, such that the left arm 11-1 and the right arm 11-2 are closed and clamped on the tongue septum fascia 200. The surgical delivery instrument is quit finally, such that the delivery instrument is disengaged from the positioning convex step/hole 11-1-1 and the positioning convex step/hole 11-2-1, and the fixing clamp 11 is released.

In this embodiment, the mandible fixer 3 is a threaded bone nail 31. The excess force protection mechanism 4 is disposed at the end part of the bone nail 31. The excess force protection mechanism 4 includes an elastic mechanism 41, a housing 42 and a slide block 43. The elastic mechanism 41 is a spiral spring made from elastic titanium metal. A through hole 42-1 and a positioning groove 42-2 are provided on the housing 42. The elastic mechanism 41 and the slide block 43 are mounted in the housing 42. The slide block 43 is mounted at one end, close to the bone nail 31, of the elastic mechanism 41. A mounting groove 31-1 and a positioning sheet 31-2 matching the housing 42 are further provided at the end part of the mandible fixer 3. After the housing 42 is inserted into the mounting groove 31-1, the positioning sheet 31-2 is inlaid in the positioning groove 42-2, and the excess force protection mechanism 4 is connected to the mandible fixer 3. The mandible fixer 3 is further provided with a line fixing mechanism 33 connected to the pull line 2. The line fixing mechanism 33 is a metal line fixing head 33-1 capable of riveting and fixing the pull line 2. One end, connected to the mandible fixer 3, of the pull line 2 sequentially passes through the through hole 42-1 of the housing 42, a center hole of the elastic mechanism 41 and a center hole of the slide block 43, and then is riveted to the line fixing head 33-1, with reference to FIG. 4-2.

With reference to FIG. 1 to FIG. 4-3, when a lower edge of the mandible 300 is selected as an entry point for implanting the implanted tongue septum fascia retraction device of the present application, a clinical surgery process is as follows:
first step: transversely incising the skin and subcutaneous tissues in the center of a front lower edge of the mandible along dermatoglyph under local anesthesia or general anesthesia;
second step: separating, after hemostasis, the subcutaneous tissues, and separating left and right musculus geniohyoideus 400 and left and right musculus genioglossus 500 along a center line using a pair of hemostatic forceps or other separators, the longitudinally extending white tongue septum fascia 200 being seen at a junction of the left and right musculus genioglossus 500, wherein in order to facilitate mounting of the fascia fixer 1, it is suggested that the white tongue septum fascia 200 having a length of about 4 cm is separated;
third step: putting the fascia fixer 1 connected with the pull line 2 to the tongue septum fascia 200 through an incision at the lower edge of the mandible 300 using a special tool;
fourth step: fixing the implanted tongue septum fascia retraction device 100 to the tongue septum fascia 200 by means of the fixing clamp 11 on the fascia fixer 1;
fifth step: fixing the mandible fixer 3 to the mandible 300 by means of the bone nail 31;
sixth step: adjusting the tightness of the pull line, cutting the pull line 2 off to a proper length, enabling the pull line 2 to sequentially pass through the through hole 42-1 of the housing 42, the center hole of the elastic mechanism 41 and the center hole of the slide block 43, riveting the pull line 2 to the line fixing head 33-1, putting the line fixing head 33-1 to a position, above the slide block 43, in the housing 42, after the housing 42 is inserted into the mounting groove 31-1, inlaying the positioning sheet 31-2 in the positioning groove 42-2, connecting the excess force protection mechanism 4 to the mandible fixer 3, and finally, suturing the incision, so as to complete implantation of the implanted tongue septum fascia retraction device 100.

When the implanted tongue septum fascia retraction device of the present application is selected to be implanted from an oral cavity, the implantation method includes:
first step: transversely incising the mucosa at lower gingival sulcus of the lower lip under local anesthesia or general anesthesia;
second step: separating and exposing front bones of the mandible, and drilling a through hole from a lower edge of the mandible 300 to a center point of a lower tooth socket using a tool;
third step: putting, after hemostasis, the fascia fixer 1 connected with the pull line 2 to the tongue septum fascia 200 through the through hole using a special tool;
fourth step: fixing the implanted tongue septum fascia retraction device 100 to the tongue septum fascia 200 by means of the fixing clamp 11 on the fascia fixer 1;
fifth step: fixing the mandible fixer 3 to the mandible 300 by means of the bone nail 31;
sixth step: adjusting the tightness of the pull line, cutting the pull line 2 off to a proper length, enabling the pull line 2 to sequentially pass through the through hole 42-1 of the housing 42, the center hole of the elastic mechanism 41 and the center hole of the slide block 43, riveting the pull line 2 to the line fixing head 33-1, putting the line fixing head 33-1 to a position, above the slide block 43, in the housing 42, after the housing 42 is inserted into the mounting groove 31-1, inlaying the positioning sheet 31-2 in the positioning groove 42-2, connecting the excess force protection mechanism 4 to the mandible fixer 3, and finally, suturing the incision, so as to complete implantation of the implanted tongue septum fascia retraction device 100.

After the implanted tongue septum fascia retraction device 100 is implanted, the tongue septum fascia 200 is retracted forwards by means of the pull line 2, such that the falling tongue base may be pulled forwards, the obstructed upper airway is opened, and the aim of treating snoring and OSA caused by glossoptosis is fulfilled.

### Embodiment 2: a fixing hook type implanted tongue septum fascia retraction device of the present application

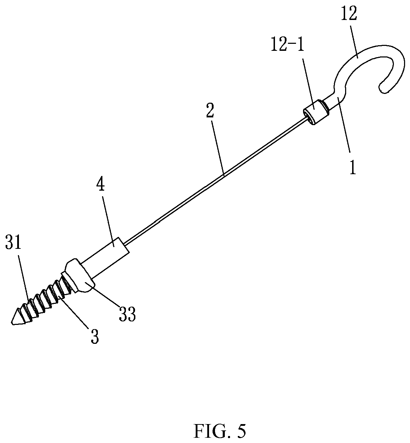

With reference to FIG. 5 to FIG. 5-2, the difference between this embodiment and Embodiment 1 lies in that the fascia fixer 1 in this embodiment is a fixing hook 12. In this embodiment, the fixing hook 12 is a C-shaped hook, made from a medical titanium metal wire, the diameter of the C-shaped fixing hook 12 is larger than 1 mm, and the C-shaped fixing hook 12 is usually made from a titanium metal wire having a diameter of 2.5 mm. The fixing hook 12 may conveniently hook the tongue septum fascia 200, thus avoiding pressure exerted on the tongue septum fascia 200 by the fixing clamp 11 in Embodiment 1. Moreover, the fixing hook 12, hooking the tongue septum fascia 200, may flexibly move so as to be suitable for irregular operation of the tongue body.

In this embodiment, a line fixing mechanism 12-1 connected to the pull line 2 is disposed at the tail part of the fixing hook 12, the line fixing mechanism 12-1 including a line fixing head 12-1-1 and a housing 12-1-2. The housing 12-1-2 is connected to the tail part of the fixing hook 12 in a threaded manner. After passing through the through hole of the housing 12-1-2, a line end at one end, connected to the fixing hook 12, of the pull line 2 is riveted and fixed to the line fixing head 12-1-1.

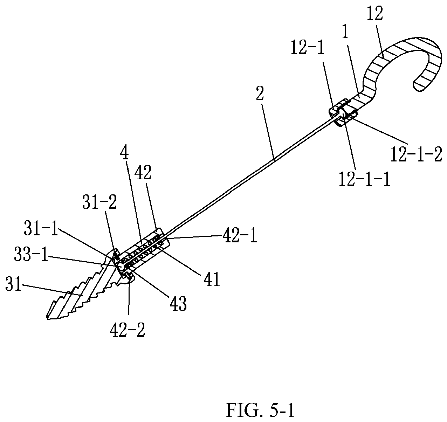

In this embodiment, the connecting manner between the pull line 2 and the mandible fixer 3 is the same as that in Embodiment 1, with reference to FIG. 5-1.

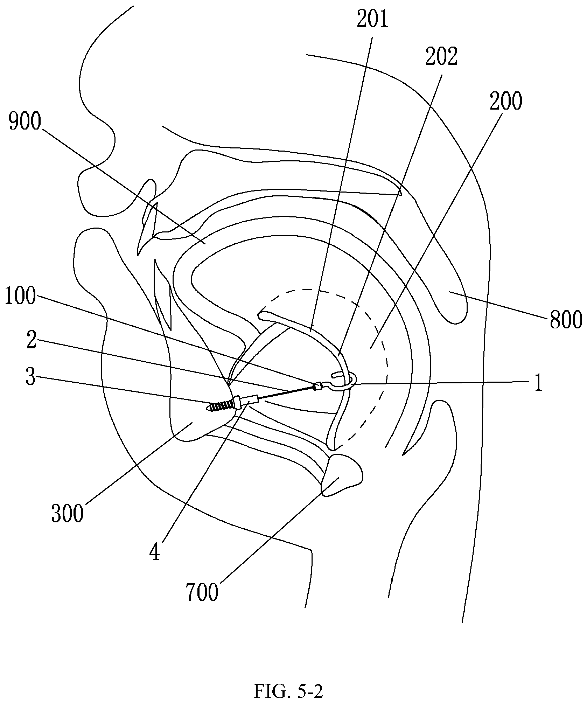

In a clinical surgery, when the implanted tongue septum fascia retraction device 100 is implanted, the fascia fixer 1 is put to the tongue septum fascia 200 through the incision at the lower edge of the mandible 300, and then the tongue septum fascia 200 passes through an opening of the fixing hook 12, such that the fixing hook 12 of the fascia fixer 1 may be connected to the tongue septum fascia 200. The tongue septum fascia 200 is pulled forwards by means of the pull line 2, such that the falling tongue base may be pulled forwards, the obstructed upper airway is opened, and the aim of treating snoring and OSA is fulfilled, with reference to FIG. 5-2.

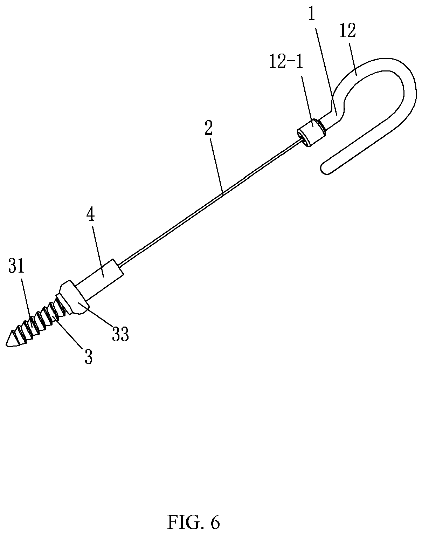

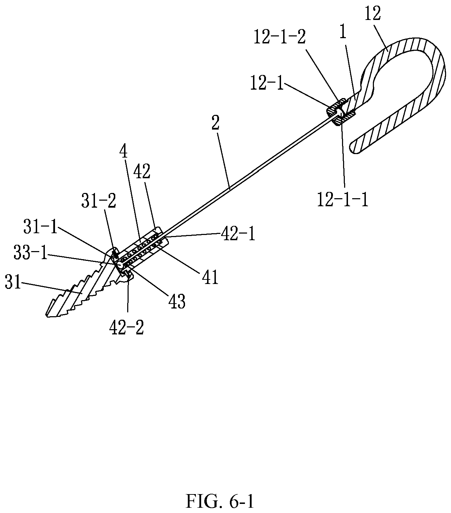

To prevent the fixing hook 12 from slipping out of the tongue septum fascia 200, the shape of the fixing hook 12 may also be designed as a U shape, with reference to FIG. 6 and FIG. 6-1, and the shape may also be designed as other more complicated shapes.

### Embodiment 3: a fixing ring type implanted tongue septum fascia retraction device of the present application

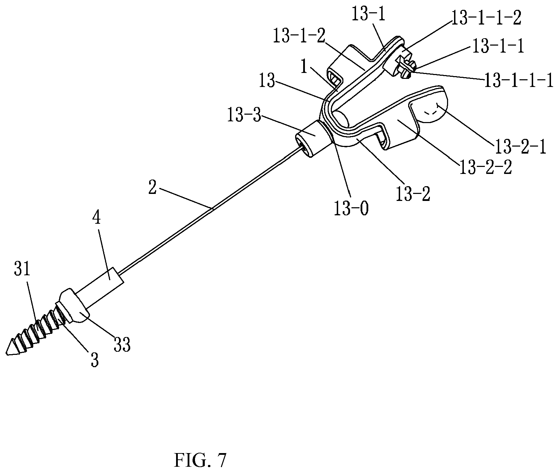

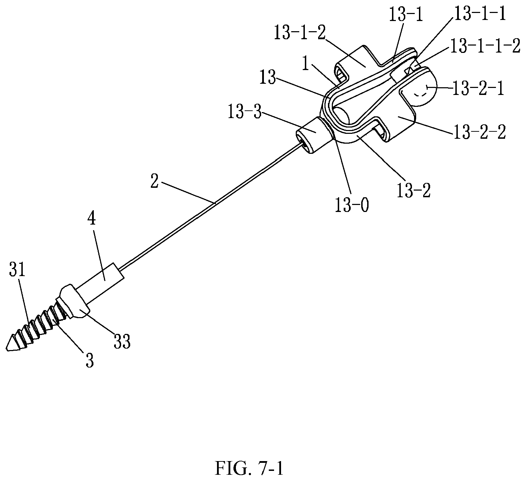

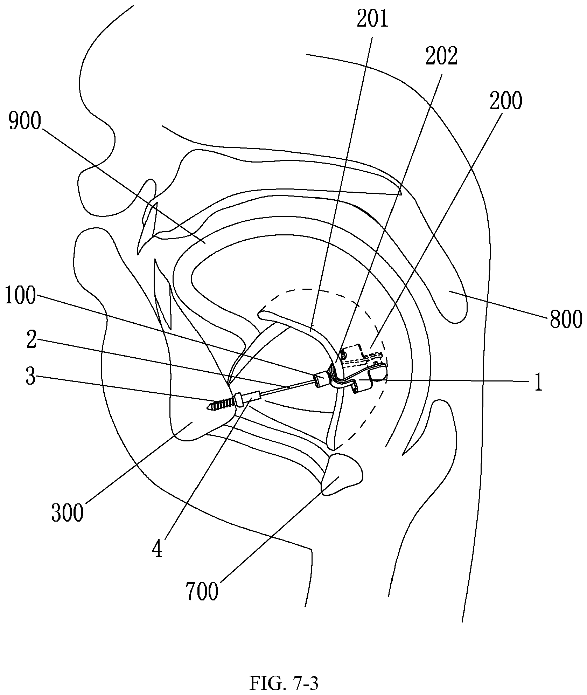

With reference to FIG. 7 to FIG. 7-3, the difference between this embodiment and Embodiment 1 lies in that the fascia fixer 1 in this embodiment is a fixing ring 13.

The fixing ring 13 includes a left arm 13-1 and a right arm 13-2, a positioning hole 13-2-1 is provided on the right arm 13-2, a puncture rod 13-1-1 is disposed on the left arm 13-1, and the puncture rod 13-1-1 passes through the tongue septum fascia 200 and then matches the positioning hole 13-2-1, so as to form a closed ring structure, with reference to FIG. 7 to FIG. 7-3.

In this embodiment, a groove hole 13-1-1-1 is provided in the puncture rod 13-1-1, thus making it convenient to insert the puncture rod 13-1-1 into the positioning hole 13-2-1 to be inlaid and fixed. The puncture rod 13-1-1 is further provided with a positioning convex step 13-1-1-2, the height (usually around 1 mm) of the positioning convex step 13-1-1-2 being slightly larger than the thickness of the tongue septum fascia 200, thus preventing the tongue septum fascia 200 from being over-pressed after the left arm 13-1 and the right arm 13-2 of the fixing ring 13 are closed, and maintaining the biological activity of the tongue septum fascia 200.

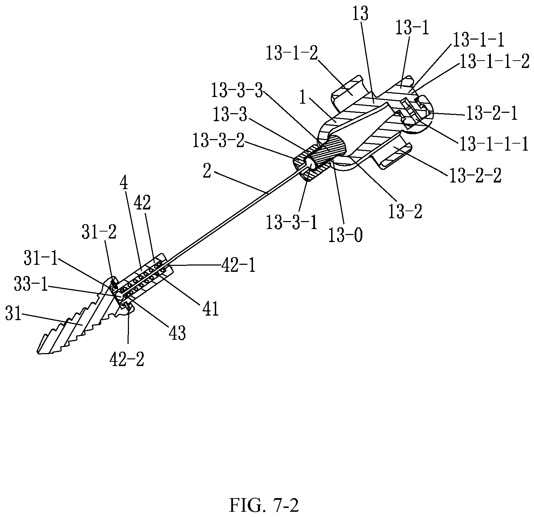

A through hole 13-0 is provided at a junction of the left arm 13-1 and the right arm 13-2 of the fixing ring 13, namely the tail end of the fixing ring 13. A line connecting mechanism 13-3 connected to the pull line 2 is disposed at the tail end of the fixing ring 13. The line connecting mechanism 13-3 includes a line fixing head 13-3-1, a housing 13-3-2 and a connecting bolt 13-3-3. The connecting manner between the fixing ring 13 and the pull line 2 is similar to that in Embodiment 1, with reference to FIG. 7-2 and FIG. 4-2.

In this embodiment, the connecting manner between the pull line 2 and the mandible fixer 3 is the same as that in Embodiment 1, with reference to FIG. 7-2 and FIG. 4-2.

A positioning convex step/hole 13-1-2 and a positioning convex step/hole 13-2-2 are provided on the left arm 13-1 and the right arm 13-2 respectively and configured to match a special surgical delivery instrument and perform positioning. The fixing ring 13 is kept in an open state, after passing through a tongue transverse fascia 201, the left arm 13-1 and the right arm 13-2 are placed on two sides of the tongue septum fascia 200, and the tongue septum fascia 200 is kept between the left arm 13-1 and the right arm 13-2, and then matches the special surgical delivery instrument, such that the left arm 13-1 and the right arm 13-2 are closed, the puncture rod 13-1-1 passes through the tongue septum fascia 200 and then matches the positioning hole 13-2-1, and a closed ring structure is formed. The surgical delivery instrument is quit finally, such that the delivery instrument is disengaged from the positioning convex step/hole 13-1-2 and the positioning convex step/hole 13-2-2, and the fixing ring 13 is released.

In a clinical surgery, when the implanted tongue septum fascia retraction device 100 is implanted, after the fascia fixer 1 is put to the tongue septum fascia 200 through the incision at the lower edge of the mandible 300, the puncture rod 13-1-1 passes through the tongue septum fascia 200 and matches the positioning hole 13-2-1, a closed ring structure is formed, the fixing ring 13 of the fascia fixer 1 is connected to the tongue septum fascia 200, and the tongue septum fascia 200 is pulled forwards by means of the pull line 2, such that the falling tongue base may be pulled forwards, the obstructed upper airway is opened, and the aim of treating snoring and OSA is fulfilled.

In this embodiment, only the fixing ring 13 having a rectangular ring closed structure is listed. Closed structures in other shapes such as a circular ring closed structure, an elliptical ring closed structure and a polygonal ring closed structure will not depart from the protection scope of the present application.

### Embodiment 4: an implanted tongue septum fascia retraction device having an excess force protection mechanism disposed at the end of a fascia fixer of the present application

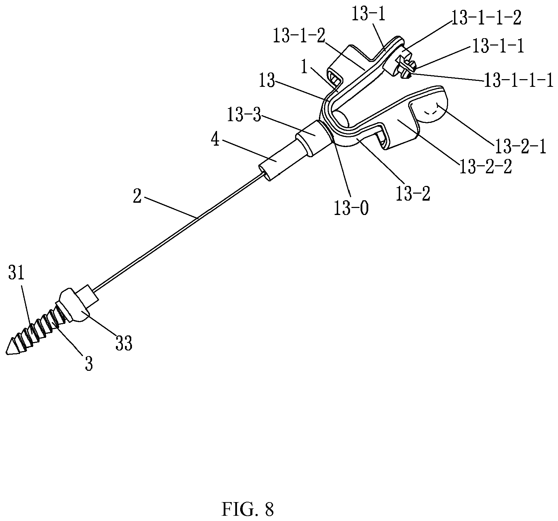

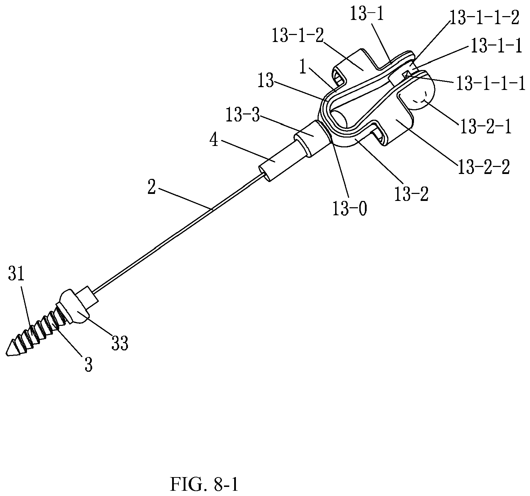

With reference to FIG. 8 to FIG. 8-2, the difference between this embodiment and Embodiment 3 lies in that the excess force protection mechanism 4 in this embodiment is disposed at the end of the fascia fixer 1. The excess force protection mechanism 4 is disposed at the end part of the fixing ring 13. The excess force protection mechanism 4 includes an elastic mechanism 41, a housing 42 and a slide block 43. The elastic mechanism 41 is a spiral spring made from elastic titanium metal. A through hole 42-1 is provided on the housing 42. The elastic mechanism 41 and the slide block 43 are mounted in the housing 42. The slide block 43 is mounted at one end, close to the fixing ring 13, of the elastic mechanism 41.

A through hole 13-0 is provided at the tail part of the fixing ring 13, and the fixing ring 13 is connected to the housing 42 of the excess force protection mechanism 4 by means of a connecting bolt 13-3-3 of the line connecting mechanism 13-3 in a threaded connection manner.

After being riveted to a metal line fixing head 31-3, the pull line 2 is inlaid in a mounting groove 31-1 at the tail part of the bone nail 31.

Embodiment 5: an implanted tongue septum fascia retraction device having an excess force protection mechanism disposed in the middle of a pull line of the present application

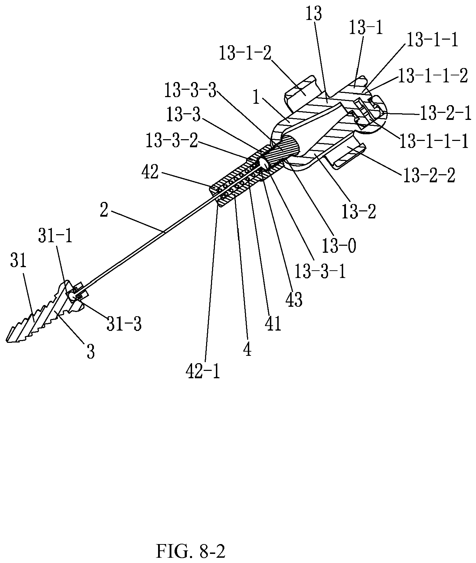

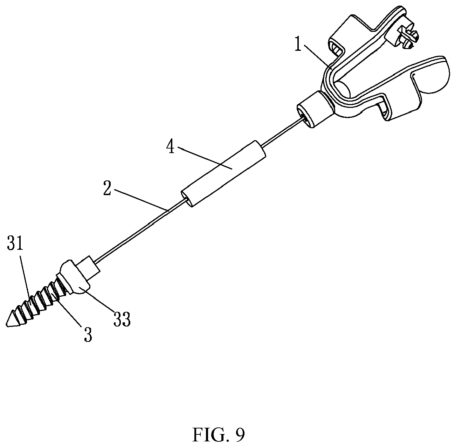

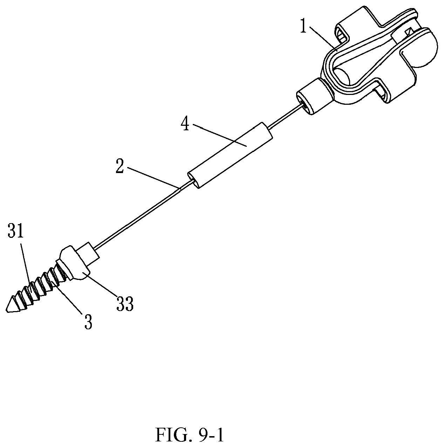

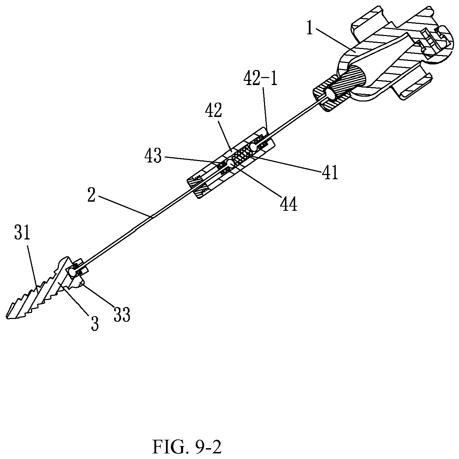

With reference to FIG. 9 to FIG. 9-2, the difference between this embodiment and Embodiment 3 lies in that the excess force protection mechanism 4 in this embodiment is disposed in the middle of the pull line 2. The excess force protection mechanism 4 includes an elastic mechanism 41, a housing 42, a slide block 43 and a line fixing head 44. The elastic mechanism 41 is a close-packed spiral spring made from elastic titanium metal. A through hole 42-1 is provided on the housing 42. The elastic mechanism 41 and the slide block 43 are mounted in the housing 42. Metal spring wires at two ends of the elastic mechanism 41 are riveted to the pull line 2 by means of the metal line fixing head 44 separately. The slide block 43, the elastic mechanism 41 and the line fixing head 44 may move in the housing 42. The connecting manner between one end of the pull line 2 and the fixing ring 13 is similar to that in Embodiment 3, with reference to FIG. 9-2 and FIG. 7-2. The connecting manner between the other end of the pull line 2 and the bone nail 31 is similar to that in Embodiment 4, with reference to FIG. 9-2 and FIG. 8-2.

### Embodiment 6: an implanted tongue septum fascia retraction device having an adjustable mandible fixer of the present application

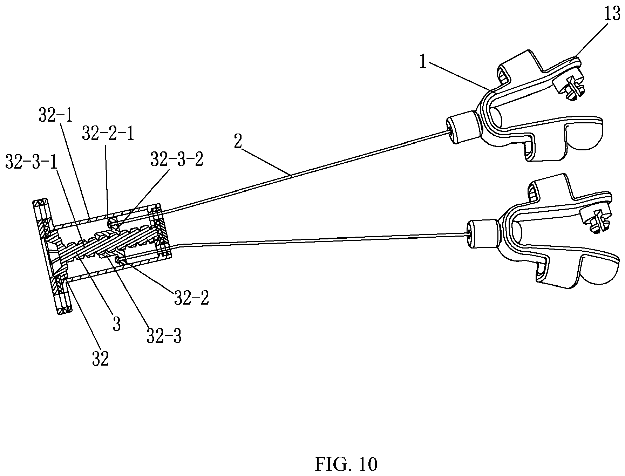

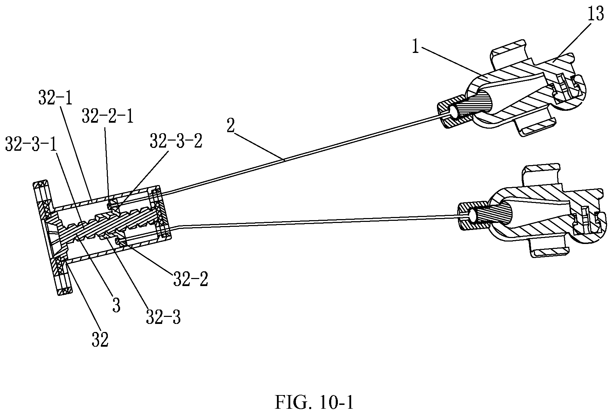

With reference to FIG. 10 to FIG. 10-2, the difference between this embodiment and Embodiment 3 lies in that the mandible fixer 3 in this embodiment is an adjustable fixing mechanism type mandible fixer 32. The fixing mechanism type mandible fixer 32 includes a housing 32-1, a pull line fixing mechanism 32-2 and a pull line tension adjusting mechanism 32-3. The pull line fixing mechanism 32-2 and the pull line tension adjusting mechanism 32-3 are mounted in the housing 32-1.

The pull line fixing mechanism 32-2 is a metal line fixing head 32-2-1 capable of riveting the pull line 2.

The pull line tension adjusting mechanism 32-3 includes a rotating screw 32-3-1 and a screw slide block 32-3-2. The rotating screw 32-3-1 is mounted on a central shaft of the housing 32-1, and by rotating the rotating screw 32-3-1, the screw slide block 32-3-2 can be driven by means of a threaded structure to reciprocate. When the pull line 2 needs to be tensioned, the rotating screw 32-3-1 may be rotated clockwise, the screw slide block 32-3-2 moves to the direction of the mandible, the distance between the fascia fixer 1 and the mandible fixer 3 is shortened, the retraction force is increased, and the retraction action on the tongue septum fascia is enhanced. Otherwise, when the pull line 2 needs to be loosened, the rotating screw 32-3-1 is rotated anticlockwise, the screw slide block 32-3-2 moves to the direction of the tongue base, the distance between the fascia fixer 1 and the mandible fixer 3 is prolonged, the retraction force is decreased, and the retraction action on the tongue septum fascia is reduced.

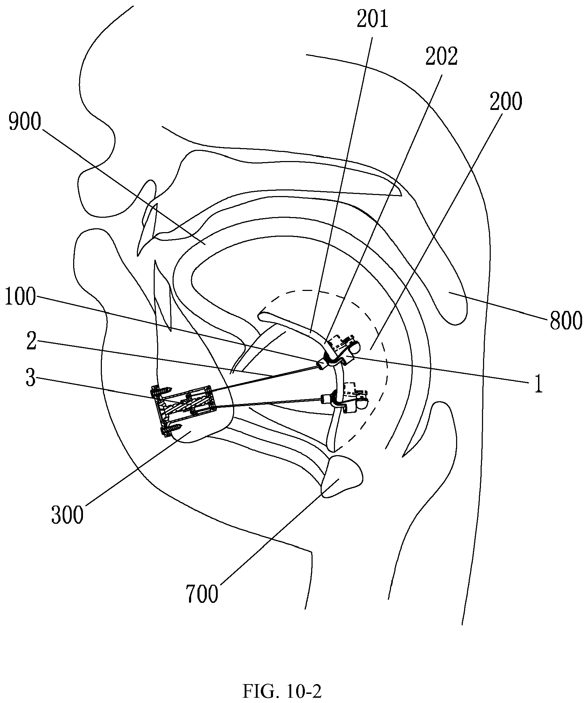

During clinical use, a through hole running through the mandible 300 is provided at a proper part of the mandible 300, and then the mandible fixer 3 is placed in the provided through hole and fixed using a bone nail, so as to complete mounting of the mandible fixer 3 on the mandible 300. With reference to Embodiment 3, the fixing ring 13 of the fascia fixer 1 is sequentially mounted at different parts of the tongue septum fascia 200 needing to be retracted. Then, the other end of the pull line 2 is connected to the mandible fixer 3, so as to complete mounting of the implanted tongue septum fascia retraction device of the present application. In this embodiment, the mandible fixer 3 may pull a plurality of fascia fixers 1 by means of a plurality of pull lines 2. Thus, the retraction range of the tongue base portion may be expanded, and a better glossopharyngeal part airway opening effect is obtained, with reference to FIG. 10-2.

### Embodiment 7: an implanted tongue septum fascia retraction device having a suspended mandible fixer of the present application

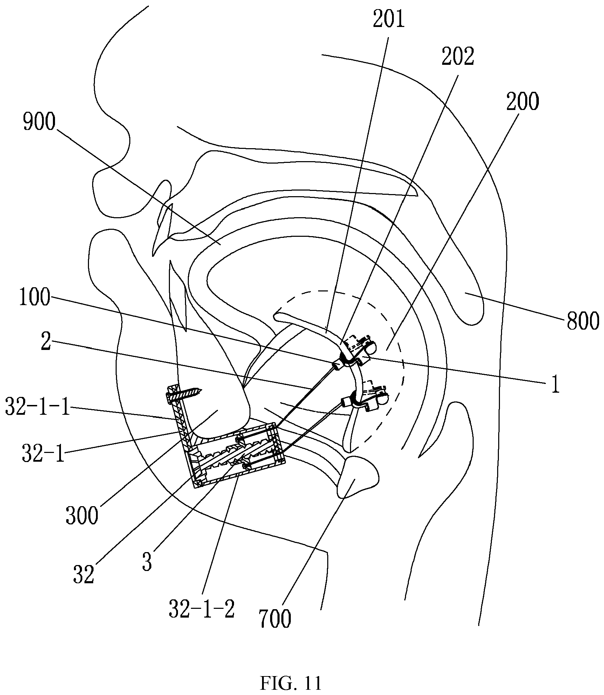

With reference to FIG. 11, in this embodiment, different from Embodiment 6, the housing 32-1 of the fixing mechanism type mandible fixer 32 is of an L-shaped structure. This fixing mechanism type mandible fixer 32 of an L-shaped structure may be suspended at the lower part of the mandible 300, and the tongue septum fascia 200 is retracted.

An end cover 32-1-1 of the housing 32-1 may be nailed at the front end of the mandible 300 using a bone nail, and a main body 32-1-2 of the housing 32-1 may be suspended below the mandible 300.

### Embodiment 8: an implanted tongue septum fascia retraction device having an adjustable mandible fixer including an excess force protection mechanism of the present application

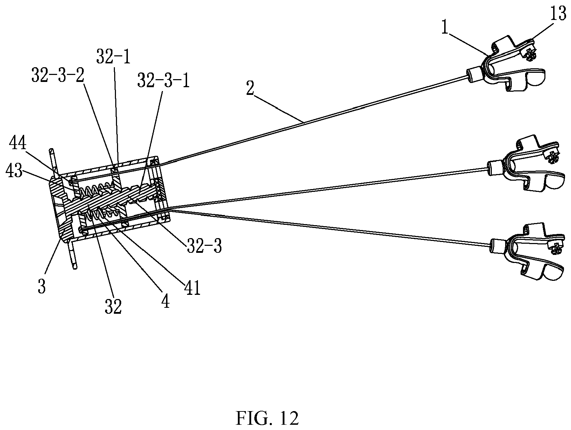

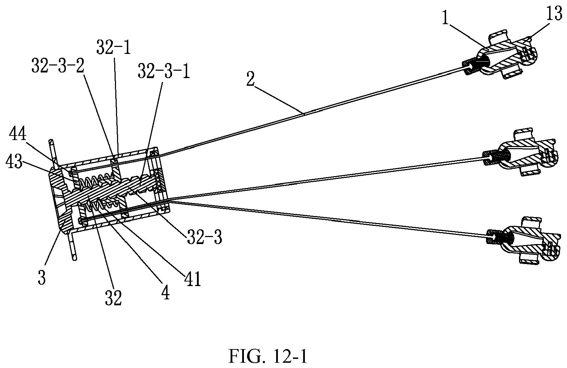

With reference to FIG. 12 to FIG. 12-1, the difference between this embodiment and Embodiment 6 lies in that an excess force protection mechanism 4 is further disposed in a housing 32-1 of the mandible fixer 3 in this embodiment. The excess force protection mechanism 4 includes an elastic mechanism 41, a slide block 43 and a line fixing head 44. The slide block 43 is mounted at one end, close to the mandible, of the elastic mechanism 41, and the pull line 2 sequentially passes through a through hole of the housing 32-1 of the mandible fixer 3, a through hole of the screw slide block 32-3-2 and a through hole of the slide block 43 of the excess force protection mechanism 4, and then is riveted to the line fixing head 44. In case of sleep apnea, the implanted tongue septum fascia retraction device of the present application has a sufficient tension, resists the tension generated on the tongue base portion during inspiration, keeps the airway of the glossopharyngeal portion open, and fulfills the aim of treating snoring and OSA. During saliva swallowing or other swallowing activities, the force of backward movement of the tongue body is usually large. When the force generated by the swallowing activities is larger than the deformation force of the elastic mechanism 41, the elastic mechanism 41 deforms, a buffer function is achieved, the comfort may be improved, and the tongue tissues are protected from being injured accidentally.

It should be noted that, the structures disclosed and described in the present application may be replaced by other structures with the same effect, and the embodiments described in the present application are not intended to limit the present application. Though the preferred embodiments of the present application have been introduced and described in the specification, persons skilled in the art should know that these embodiments are merely described by way of example, and persons skilled in the art may make various changes, improvements, and replacements without departing from the present application. Therefore, the protection scope of the present application should be defined in accordance with the spirit and scope of the appended claims of the present application.

## Claims

1. An implanted tongue septum fascia retraction device, wherein
A, the implanted tongue septum fascia retraction device (100) comprises: a fascia fixer (1), a pull line (2) and a mandible fixer (3), the fascia fixer (1) being a fixing clamp (11) capable of clamping a tongue septum fascia (200), a fixing hook (12) capable of passing through the tongue septum fascia (200) or a fixing ring (13) capable of passing through the tongue septum fascia (200), the pull line (2) being a linear object capable of being implanted into a human body for a long time, the mandible fixer (3) being a fixing mechanism capable of being fixed to a mandible (300); and
B, one end of the pull line (2) is connected to the fascia fixer (1), and the other end of the pull line (2) is connected to the mandible fixer (3).

2. The implanted tongue septum fascia retraction device according to claim 1, wherein the implanted tongue septum fascia retraction device (100) further comprises an excess force protection mechanism (4); the excess force protection mechanism (4) is disposed at the end of the mandible fixer (3), one end of the excess force protection mechanism (4) is connected to the mandible fixer (3), and the other end of the excess force protection mechanism (4) is connected to the pull line (2); or, the excess force protection mechanism (4) is disposed at the end of the fascia fixer (1), one end of the excess force protection mechanism (4) is connected to the fascia fixer (1), and the other end of the excess force protection mechanism (4) is connected to the pull line (2); or, the excess force protection mechanism (4) is disposed in the middle of the pull line (2), and two ends of the excess force protection mechanism (4) are connected to the pull line (2) separately.

3. The implanted tongue septum fascia retraction device according to claim 2, wherein the excess force protection mechanism (4) comprises an elastic mechanism (41).

4. The implanted tongue septum fascia retraction device according to claim 3, wherein the elastic mechanism (41) adopts a spring structure.

5. The implanted tongue septum fascia retraction device according to claim 4, wherein the spring structure is a spiral spring structure.

6. The implanted tongue septum fascia retraction device according to claim 2, wherein the excess force protection mechanism (4) further comprises a housing (42).

7. The implanted tongue septum fascia retraction device according to claim 6 and claim 4, wherein the elastic mechanism (41) of the excess force protection mechanism (4) is disposed in the housing (42) of the excess force protection mechanism (4).

8. The implanted tongue septum fascia retraction device according to claim 1, wherein a concave-convex engagement structure is formed between a left arm (11-1) and a right arm (11-2) of the fixing clamp (11) of the fascia fixer (1).

9. The implanted tongue septum fascia retraction device according to claim 1, wherein the fixing hook (12) of the fascia fixer (1) is a C-shaped hook or a U-shaped hook.

10. The implanted tongue septum fascia retraction device according to claim 1, wherein the fixing ring (13) of the fascia fixer (1) comprises a left arm (13-1) and a right arm (13-2), a positioning hole (13-2-1) is provided on the right arm (13-2), a puncture rod (13-1-1) is disposed on the left arm (13-1), and the puncture rod (13-1-1) passes through the fascia (200) and then matches the positioning hole (13-2-1), so as to form a closed ring structure.

11. The implanted tongue septum fascia retraction device according to claim 10, wherein the fixing ring (13) may be of a circular ring structure or an elliptical ring structure or a polygonal ring structure.

12. The implanted tongue septum fascia retraction device according to claim 1, wherein the pull line (2) is an elastic line or a non-elastic line that is implanted into a human body for a long time.

13. The implanted tongue septum fascia retraction device according to claim 1, wherein the mandible fixer (3) is a bone nail (31).

14. The implanted tongue septum fascia retraction device according to claim 1, wherein the mandible fixer (3) is a fixing mechanism (32) capable of being fixed to the mandible (300), the fixing mechanism (32) comprising a housing (32-1) and a line fixing mechanism (32-2) capable of fixing the pull line.

15. The implanted tongue septum fascia retraction device according to claim 14, wherein the fixing mechanism (32) comprises an adjusting device (32-3) capable of adjusting the tightness of the pull line (2), the adjusting device (32-3) being a threaded adjusting mechanism, a rotary shaft type adjusting mechanism or a slide block type adjusting mechanism.

16. An implantation method for implanting an implanted tongue septum fascia retraction device from a mandible, comprising:
first step: transversely incising the skin and subcutaneous tissues in the center of a front lower edge of the mandible along dermatoglyph under local anesthesia or general anesthesia;
second step: separating, after hemostasis, the subcutaneous tissues, and separating left and right musculus geniohyoideus (400) and left and right musculus genioglossus (500) along a center line using a pair of hemostatic forceps or other separators, a longitudinally extending white tongue septum fascia (200) being seen at a junction of the left and right musculus genioglossus (500);
third step: putting a fascia fixer (1) connected with a pull line (2) to the tongue septum fascia (200) through an incision at the lower edge of the mandible (300) using a special tool;
fourth step: fixing an implanted tongue septum fascia retraction device (100) to the tongue septum fascia (200) by means of a fixing clamp (11), a fixing hook (12) or a fixing ring (13) on the fascia fixer (1);
fifth step: fixing a mandible fixer (3) to the mandible (300) by means of a bone nail (31) or a fixing mechanism (32) capable of being fixed to the mandible (300); and
sixth step: connecting the pull line (2) to the mandible fixer (3), adjusting the tightness of the pull line 2, and suturing the incision, so as to complete implantation of the implanted tongue septum fascia retraction device (100).

17. An implantation method for implanting an implanted tongue septum fascia retraction device from an oral cavity, comprising:
first step: transversely incising a mucosa at lower gingival sulcus of the lower lip under local anesthesia or general anesthesia;
second step: separating and exposing front bones of a mandible, and drilling a through hole from a lower edge of the mandible (300) to a center point of a lower tooth socket (600) using a tool;
third step: putting, after hemostasis, a fascia fixer (1) connected with a pull line (2) to a tongue septum fascia (200) through the through hole using a special tool;
fourth step: fixing an implanted tongue septum fascia retraction device (100) to the tongue septum fascia (200) by means of a fixing clamp (11) or a fixing hook (12) or a fixing ring (13) on the fascia fixer (1);
fifth step: fixing a mandible fixer (3) to the mandible (300) by means of a bone nail (31) or a fixing mechanism (32) capable of being fixed to the mandible (300); and
sixth step: connecting the pull line (2) to the mandible fixer (3), adjusting the tightness of the pull line (2), and suturing the incision, so as to complete implantation of the implanted tongue septum fascia retraction device (100).
